# EUROPEAN PATENT APPLICATION

(11) **EP 3 620 110 A1**
(43) Date of publication of application: **11.03.2020**
(21) Application number: 18193227.8
(22) Date of filing: 07.09.2018
(51) Int. Cl.: A61B 6/00

(54) **AN X-RAY IMAGING DEVICE**

(71) Applicant: Koninklijke Philips N.V., 5656 AE Eindhoven (NL)
(72) Inventor: VAN PINXTEREN, Jeffrey Adrianus Wilhelmus, 5656 AE Eindhoven (NL); VAN LOON, Robertus Johannes Adrianus, 5656 AE Eindhoven (NL); VERMEULEN, Johannes Petrus Martinus Bernardus, 5656 AE Eindhoven (NL)
(74) Representative: de Haan, Poul Erik

(57) **Abstract**

The present invention relates to X-ray imaging. In order to provide increased stability for X-ray imaging devices, an X-ray imaging device is provided (10) that comprises an X-ray imaging arrangement (12) with an X-ray source (14) and an X-ray detector (16); The X-ray imaging device also comprises a support structure (18) that has a base (20) configured to be rotatably attached to a fixed part of a building structure such that the base can be rotated in relation to the fixed part. The support structure also has a C-arm (22) with two opposing ends, at which the X-ray source and the X-ray detector are mounted. The support structure further has a sliding carrier support (24) with which the C-arm is movably mounted to the base. The support structure is movably carrying the X-ray imaging arrangement with a limitation of two degrees of freedom: i) the rotatably attached base provides a first degree of freedom in a first rotational manner (26); and ii) the sliding carrier support provides a second degree of freedom such that the C-arm can be moved in relation to the sliding carrier support along an arc in a second rotational manner (28). The first rotational manner and the second rotational manner are provided transverse to each other. Further, the first rotational manner is provided around a vertical axis of rotation (30).

## Description

### FIELD OF THE INVENTION

The present invention relates to X-ray imaging, and relates in particular to an X-ray imaging device, to an X-ray imaging system and to a method for movably supporting an X-ray imaging arrangement.

### BACKGROUND OF THE INVENTION

For gaining knowledge of an object's interior, e.g. a body's interior, X-ray imaging devices are used to provide 2D and 3D image data of an object. Visual representations of a body's interior can be used to provide a physician with information e.g. during image guided therapy. Among others, 2D X-ray images are used (also called projections). These images are taken by transmission of X-radiation through the patient's body, at a certain position and orientation. Depending on the nature of the procedure, and the anatomy of the patient, various projections at different orientations are required. As an example, such images can be used to provide feedback, e.g. for a physician, during a medical procedure, such as an intervention or examination. The imaging devices are equipped with an X-ray source and an X-ray detector, which may be moved e.g. spherically with respect to a patient. Devices may contain multiple rotational joints, which are connected by long and slender structural elements to allow for large angles of rotation and to minimize obstruction to the medical procedure and staff. However, it also results in complex devices which have a large structural mass and relatively poor motion performance (quasi-static and dynamic). It has been shown that the quality of the 2D images can be degraded by vibrations of the imaging equipment with respect to each other and the patient. For example, US 6461039 B1 relates to an X-ray device with a C-arm carrying an X-ray source and an X-ray detector. The device includes a suspension device with a joint, the X-ray source and the X-ray detector being rotatable about a propeller axis which extends through the joint. In order to enable flexible movements in such an X-ray device, the position of the propeller axis can be changed in all spatial directions. Multiple 2D images, taken over a large angular range, can also be combined into 3D reconstructions (computed tomography, CT). The ability to make such reconstructions during a medical procedure is proven to be an asset for many clinical disciplines. It provides the physicians with a clearer image of complex physical structures. The use of 3D reconstructions also decreases the need for time consuming preoperative diagnostics, and postoperative check-ups. However, imperfections in 3D reconstructions can be caused by uncertainties in the source and detector positioning.

### SUMMARY OF THE INVENTION

There may thus be a need to provide increased stability for X-ray imaging devices.

The object of the present invention is solved by the subject-matter of the independent claims; further embodiments are incorporated in the dependent claims. It should be noted that the following described aspects of the invention apply also for the X-ray imaging device, for the X-ray imaging system and for the method for method for movably supporting an X-ray imaging arrangement.

According to the present invention, an X-ray imaging device is provided that comprises an X-ray imaging arrangement with an X-ray source and an X-ray detector, and that also comprises a support structure. The support structure comprises a base configured to be rotatably attached to a fixed part of a building structure such that the base can be rotated in relation to the fixed part. The support structure also comprises a C-arm with two opposing ends, at which the X-ray source and the X-ray detector are mounted. The support structure further comprises a sliding carrier support with which the C-arm is movably mounted to the base. The support structure is movably carrying the X-ray imaging arrangement with a limitation of two degrees of freedom: i) the rotatably attached base provides a first degree of freedom in a first rotational manner; and ii) the sliding carrier support provides a second degree of freedom such that the C-arm can be moved in relation to the sliding carrier support along an arc in a second rotational manner. The first rotational manner and the second rotational manner are provided transverse to each other. Further, the first rotational manner is provided around a vertical axis of rotation.

By providing only two rotational movements, but with a traverse arrangement wherein one axis is vertically oriented, an imaging device is provided that allows enough flexibility for imaging in a sufficiently large variety of directions. However, due to the limitation to two degrees of freedom, a reduction in parts is achieved plus an increase in stability. The imaging device can thus be lighter while still being sufficiently rigid. Besides easier handling due to lighter weight, a lightweight construction also implies lower costs due to less material. The support device targetedly reduces the degrees of freedom for moving and thus spatially adjusting the X-ray imaging arrangement. The movement possibilities are limited to two degrees of freedom, which preferably do not include a propeller movement of the C-arm. A propeller function of the C-arm, where e.g. the C-arm is held by the sleeve and the sleeve is rotatably mounted to allow a movement of the C-arm where the ends of the C-arm with attached X-ray source and detector rotate like the end of propeller blades, is thus not possible. The limitation to two degrees of freedom advantageously also provides less vibration and also less deflection due to the increased stability. This also leads to better X-ray image quality. Although the movement possibilities are limited, for most of the imaging routine tasks, same projection directions can be provided. The absence of a propeller rotation also decreases the number of structural components, actuators, and measuring systems.

Both the sliding carrier and the base can be manufactured from a structural material such as steel, aluminum, or carbon fiber reinforced plastics. They can be designed as a thin walled, boxed shape. This results in a stiff but lightweight structure, and allows internal placement of components (e.g. cabling, drive systems).

According to an example, the sliding carrier support is provided with a guiding section part that extends on one side of the base as an arc-segment.

According to an example, the guiding section part comprises a bearing section of several bearings. Further, the sliding movement of the C-arm along the sliding carrier support is limited to a range in which the C-arm is supported by all of the several bearings.

As an advantage, by ensuring the permanent contact with the complete set of bearings, any sudden wobble is avoided that may occur when leaving the contact with a bearing.

According to an example, the base is configured as a moveable floor base that comprises a plurality of supporting modules configured to provide support for the base in a vertical direction on a floor surface and to allow a movement of the base in horizontal direction. The moveable floor base also comprises a central bearing module configured to provide releasable fixation of the base in the horizontal plane of the floor while allowing rotational movement of the base. The moveable floor base further comprises a locking mechanism configured to provide temporal fixation of the rotational movement of the base during imaging.

According to an example, at least one of the supporting modules is temporarily lockable to prevent rotational movement during imaging.

According to an example, the central bearing module is temporarily lockable to prevent the rotational movement during imaging.

According to the present invention, also an X-ray imaging system is provided that comprises an X-ray imaging device according to one of the preceding examples and a subject support. During use, the base is arranged below or above the subject support. In an example, the base is arranged in vertical alignment with a region of the subject support in which an isocenter will be arranged, e.g. where a region of interest of an object will be arranged for imaging.

According to the present invention, also a method for movably supporting an X-ray imaging arrangement is provided. The method comprises the following steps.
- In a first step, an X-ray imaging arrangement with an X-ray source and an X-ray detector is provided. The device has a support structure with a base configured to be rotatably attached to a fixed part of a building structure such that the base can be rotated in relation to the fixed part, and a C-arm with two opposing ends, at which the X-ray source and the X-ray detector are mounted, and a sliding carrier support with which the C-arm is movably mounted to the base. The support structure is movably carrying the X-ray imaging arrangement with a limitation of two degrees of freedom: The rotatably attached base provides a first degree of freedom in a first rotational manner, and the sliding carrier support provides a second degree of freedom such that the C-arm can be moved in relation to the sliding carrier support along an arc in a second rotational manner. The first rotational manner and the second rotational manner are provided transverse to each other; and the first rotational manner is provided around a vertical axis of rotation.
- In a first sub-step of a second step, the base is rotatably moved around the first axis.
- In addition, or alternatively, in a second sub-step of the second step, the C-arm is slidingly moved within the sliding carrier support to rotatably move the C-arm around the second axis.

According to an example of the method, a dual axis movement is provided.
- For the second sub-step, the C-arm with the X-ray imaging arrangement is rotated in an initial rotation in the sliding carrier support to a first angle around the second axis of rotation as a starting position. Subsequently, the C-arm is rotated in a rotation (which rotation can also be referred to as C-arm-rotation) from this starting position to a near-neutral position in which an imaging viewing direction is approximately aligned with the first axis of rotation, and back to the starting position.
- For the first sub-step, the base is rotated in a rotation (which rotation can also be referred to as base-rotation) over an angle of approximately 180° around the first axis of rotation. These two rotation motions are synchronized such that a moment of nearly zero roll velocity around the second axis of rotation corresponds to a rotation of the base around the first axis of rotation of approximately 90°.

The actual dual axis scan comprises the movements of the two rotations, i.e. the C-arm-rotation and the base-rotation. Those movements are executed synchronously. The movement to the starting position is not necessarily combined with a rotational movement of the base.

In the given example, a 3D movement of the X-ray imaging arrangement is provided by a combined movement, in which the first and the second degree of freedom are used to different amounts.

As a result, shorter strokes, i.e. reduced required range of motion for one axis (to do a complete 3D by addressing one axis), are compensated via a usage of the respective other axis. Thus, a large 3D trajectory is provided despite the reduction to two degrees of freedom.

According to an aspect, the C-arm, or C-shaped carrier, to which the imaging equipment is attached, is rotated around two axes only to position the imaging equipment at clinically relevant projection angles. A base is connected to the fixed world by a rotational joint. By this, a rotation that features an out-of-plane movement of the carrier with respect to a base is avoided. As an effect, the torsional deflection of the carrier due to gravity is eliminated. This reduces the misalignment of the imaging equipment, and causes it to be more constant over the device's range of motion. The amount of excess radiation coverage on the edges of the detector can therefore be decreased, which reduces unnecessary radiation dose for both patient and staff. Space on the sides of the carrier can be used for the structure of the base. Furthermore, compact structural components are possible, which have a good stiffness to mass ratio. The system mass can thus be reduced, which also means benefits in cost of production, transport and installation. This increases the relevant second moments of area of the base. The structural material of the base is therefore more efficiently used to resist the (now mainly in-plane) static and dynamic loads exerted by the carrier. This contributes to a lightweight design, and improved dynamic performance (image quality). Also, the building's structure to which the device is mounted, and which should be able to support this mass, can be less massive. Hence, requirements on the strength and stiffness of a medical room's floor or ceiling, for example, are reduced. The good (large) stiffness to mass ratio of the system's construction leads to improved motion performance. Vibrations between the X-ray source, detector, and patient, that would deteriorate image quality, are minimized. By avoiding any out-of-plane rotation, gravity cannot cause an unfavorable combination of bending and torsional deformation of the carrier. As a result, orientation-dependent misalignment of the imaging equipment is reduced. This allows decreased radiation dose for both the patient and medical staff.

As indicated above, according to an aspect, a device is proposed which does not feature an out-of-plane rotation of the carrier and imaging equipment with respect to the base. This allows the base to be constructed with a higher stiffness to mass ratio, and eliminates torsional deformation of the carrier due to gravity. The proposed device therefore has the following advantages: The device can be constructed at a significantly lower mass (∼500 vs ∼1200 kg), which allows the cost of production, transport, and installation to be lowered. The reduced system mass leads to reduced installation requirements. This allows the device to be installed in a greater variety of medical rooms. Deformation of the carrier due to gravitational load is reduced to bending only. This leads to less misalignment of the imaging equipment, which allows unnecessary radiation dose to be reduced. Since a near horizontal propeller axis is not provided, the section of the base which guides the carrier can be constructed close to fixed world (floor or ceiling). In an example, this section is placed close to the floor, and to the floor mounted rotational joint. This results in a compact design of the base, with a shortened path of force transmission, and reduced device inertia with respect to the rotational joint. The device also has improved dynamic behavior, which results in better image quality.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows a perspective view of an example of an X-ray imaging device in the context of an X-ray imaging system including the X-ray imaging device and a subject support.
Figs. 2a, 2b and 2c show different movement possibilities of the X-ray imaging device of Fig. 1 in schematic top views.
Figs. 3a and 3b show perspective views of exemplary postures of the X-ray imaging device of Fig. 1 for different projection angles.
Fig. 4a shows a perspective view of a starting posture for a roll scan of the X-ray imaging device of Fig. 1, and Fig. 4b shows a perspective view of an end posture of the roll scan.
Fig. 5 shows basic steps of an example of a method for movably supporting an X-ray imaging arrangement.
Fig. 6 shows steps of a further example of the method of Fig. 5.
Fig. 7a shows a begin posture of a dual axis 3D scan, Fig. 7b shows a middle posture of the dual axis 3D scan and Fig. 7c shows an end posture of the dual axis 3D scan.
Fig. 8 shows an example of a movable base of the X-ray imaging device of Fig. 1.
Fig. 9 shows a top view of the base of Fig. 8.
Fig. 10 shows a vertical cross section of the base of Fig. 9.
Fig. 11 shows an example of a ceiling-attached X-ray imaging device.

### DETAILED DESCRIPTION OF EMBODIMENTS

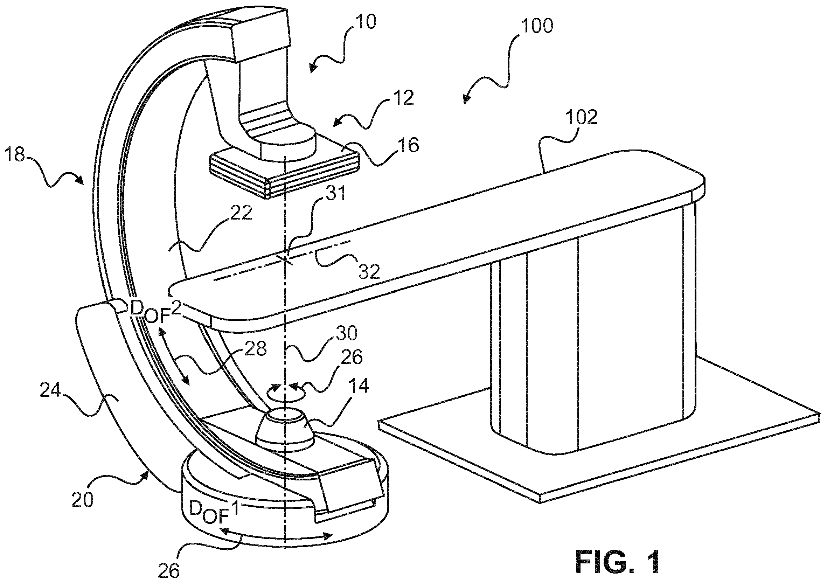

Fig. 1 shows a perspective view of an example of an X-ray imaging device 10. The X-ray imaging device 10 comprises an X-ray imaging arrangement 12 with an X-ray source 14 and an X-ray detector 16. The X-ray imaging device 10 further comprises a support structure 18. The support structure 18 comprises a base 20 configured to be rotatably attached to a fixed part of a building structure such that the base 20 can be rotated in relation to the fixed part. The support structure 18 comprises a C-arm 22 with two opposing ends, at which the X-ray source 14 and the X-ray detector 16 are mounted. The support structure 18 further comprises a sliding carrier support 24 with which the C-arm 22 is movably mounted to the base 20. The support structure 18 is movably carrying the X-ray imaging arrangement 12 with a limitation of two degrees of freedom: Firstly, the rotatably attached base 20 provides a first degree of freedom *D_{OF}1* in a first rotational manner, indicated by a double arrow 26.

Secondly, the sliding carrier support 24 provides a second degree of freedom *D_{OF}2* such that the C-arm 22 can be moved in relation to the sliding carrier support 24 along an arc in a second rotational manner, as indicated by a further double arrow 28. The first rotational manner and the second rotational manner are provided transverse to each other. Further, the first rotational manner is provided around a vertical axis of rotation 30.

In an example, the first rotational manner is provided around a first axis of rotation (i.e. a first rotational axis), and the second rotational manner is provided around a second axis of rotation (i.e. a second rotational axis). The first axis of rotation and the second axis of rotation are transverse to each other.

In an example, the first and the second rotation manner are essentially perpendicular to each other. The term "essentially perpendicular to each other" refers to an arrangement in the range of about +/- 15° from 90°, such as maximum +/- 10° from 90°, or +/- 5° from 90°.

The first rotational manner allows the horizontal axis to be aligned with the longitudinal centerline of a patient table. It is not necessarily aligned at all times.

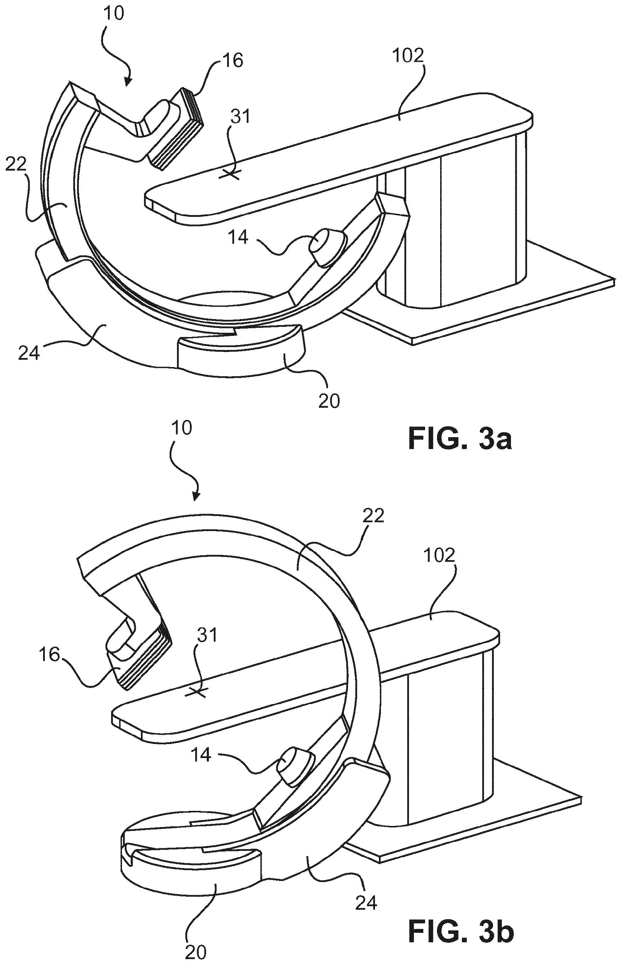

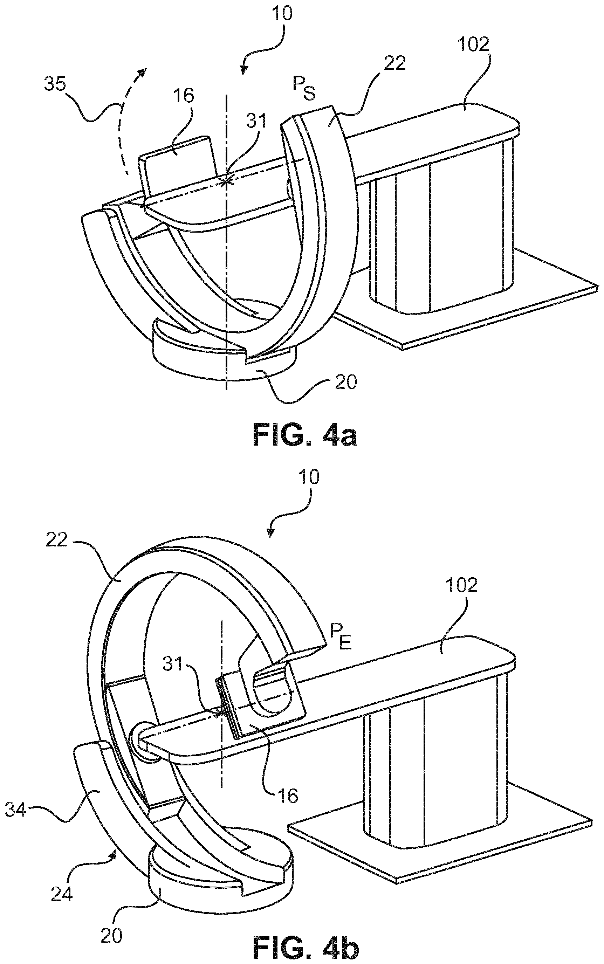

The sliding carrier support provides a curved linear movement of the C-arm along the arc shaped guide such that a roll motion is achieved. Thus, a rotation around a nominally vertical axis can be combined with a roll motion (see Figs. 3a-3b and Figs. 4a-4b).

In an example, the first rotational manner is provided around a first axis of rotation and the second rotational manner is provided around a second axis of rotation.

For example, an isocenter 31 is formed at an intersection of the first and the second axis of rotation. The roll movement provides for the first degree of freedom. It features an in-plane rotation of the carrier around the horizontal axis. This has the advantage of taking up minimal space when only a single degree of freedom rotation is required.

In an example, shown as an option, the first rotational manner and the second rotational manner are arranged essentially perpendicular to each other. As a further option, the second rotational manner is provided around a horizontal axis of rotation 32.

In another option, a rotational degree of freedom is provided for the X-ray detector and/or collimation device around the centerline of the X-ray bundle.

The term "support structure" relates to those parts that are provided to hold or mount the imaging equipment, i.e. to hold the X-ray source and the X-ray detector during imaging. The support structure can thus also be referred to a mounting arrangement or holding arrangement.

The term "base" relates to the part of the support structure that provides a connection, or coupling to the structure of the building. The base supports the X-ray source and the X-ray detector with respect to a part of the building structure.

In an example, the base is rotated in relation to the fixed point of the building structure.

The term "C-arm" relates to a supporting component that is formed like a "C" with a middle section that is shaped like an arc, or part of a circle, and which middle section has two opposing ends to which the X-ray source and the X-ray detector are mounted. The C-arm, or C-arc, can also have a polygonal shape, when a holding section is provided that allows the sliding movement in relation to the sliding carrier support in a rotational manner.

The C-arm has a length such that, in relation to an iso-center, a range of approximately 150° to 220° are covered, for example 165° to 180°.

The C-arm is also referred to as C-shaped carrier arc.

The C-arm is directly mounted to the base by the carrier support.

The term "sliding carrier support" relates to a holding device with which the C-arm's middle section is mounted and movably fixed to the base. The holding device thus carries the C-arm and provides support in form of transferring the physical loads from the X-ray source and the X-ray detector and the C-arm itself. The support is provided such that a certain movement is possible, namely the sliding movement. The carrier support allows a sliding movement in a sleeve-type manner.

The sliding carrier support is provided as a sleeve forming an arc-shaped sliding support for the C-arm carrier. The sliding carrier support provides the second degree of freedom such that the C-arm as carrier of the X-ray imaging arrangement can be moved in relation to the base along the arc in a second rotational manner. The X-ray imaging device is thus free of any supporting or cantilevering arms, except for the C-arm itself.

Fig. 1 also shows as an option an X-ray imaging system 100 that comprises a subject support 102, for example a patient table adjustable e.g. in height, inclination and horizontal rotation. The X-ray imaging system 100 also comprises an example of the X-ray imaging device 10 as described above and below in further examples. During use, the base 20 is arranged below the subject support 102. In another option, the base 20 is arranged above the subject support 102, when the base is mounted to a ceiling.

It is noted that even though the following figures also show the subject support 102, the system 100 comprising the X-ray imaging device 10 together with the subject support 102 is shown as an option only. The X-ray imaging device 10 is thus also provided by itself, i.e. without the subject support 102.

The "subject" may also be referred to as individual. The "subject" may further also be referred to as patient, although it is noted that this term does not indicate whether any illness or disease is present with the subject.

The subject support may be provided as a subject table (or patient table), on which the subject can be arranged during the procedure, examination or intervention.

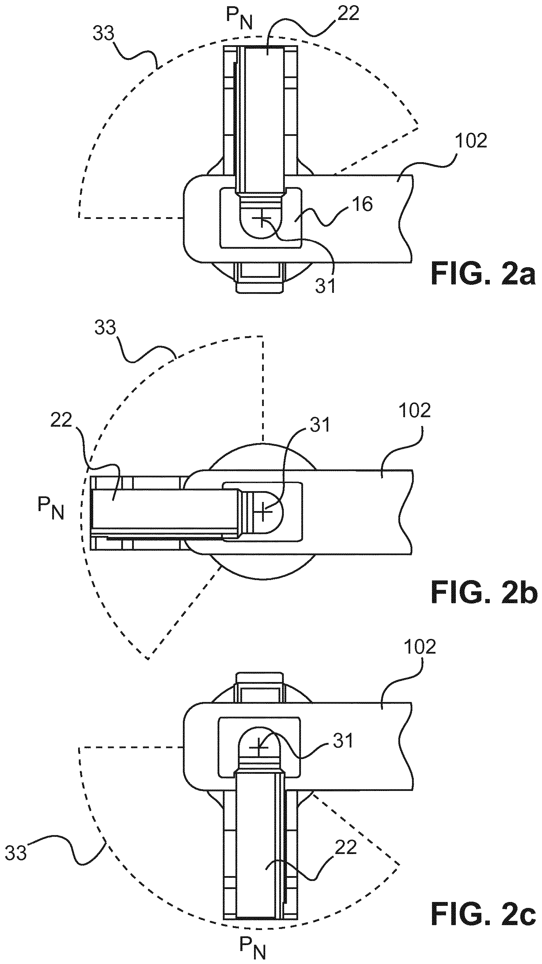

Figs. 2a, 2b and 2c show the rotation of the base 20 around the vertical axis 30 that forms one of the two required degrees of freedom. Fig. 2a shows the device 10 with a so-called neutral position *P_{N}* at a so-called nurse side of the patient table, i.e. the patient support 102. Fig. 2b shows the device 10 with the neutral position *P_{N}* at a so-called head end of the patient support 102. Fig. 2c shows the device 10 with the neutral position *P_{N}* at a so-called doctor side of the patient support 102.

As can be seen, this allows the user to choose for the neutral position *P_{N}* of the X-ray imaging device 10 at either side or at the head end of the patient support 102. The X-ray imaging device 10 can be provided with its neutral position at the nurse side, the head end, and the doctor side of the patient table. From this neutral position *P_{N}*, clinically relevant projections angles can be accessed by rotating the base 20 around the vertical axis 30 over an angle 33, indicated with hashed lines. This angle, or angular range, can be seen as a working region of the X-ray imaging device 10, and is specific to the chosen neutral position *P_{N}.*

3D reconstructions can also be generated from a set of 2D X-ray images. These images are taken while rotating the imaging equipment around the patient's longitudinal axis, over a certain angular range. In an example, the X-ray imaging device 10 is able to capture a set of 2D images in a number of ways.

Figs. 3a and 3b show the X-ray imaging device 10 in relation to the patient support 102 in exemplary projection angles. The position shown in Fig. 3a corresponds to the neutral position at head end, or nurse side of the patient support. The position shown in Fig. 3b corresponds to the neutral position at doctor side of patient table. As can be seen, the base 20 is rotated around the vertical axis of rotation 30 and the C-arm 22 is also rotated in a semicircular manner (by sliding movement in the sliding carrier support 24) around the second axis of rotation.

In an example, a 3D roll scan is provided. This involves a rotation of the carrier including the imaging equipment around the horizontal axis. It can be carried out from a neutral position at either side of the patient table.

Fig. 4a shows a perspective view of a starting posture *P_{S}* for a roll scan of the X-ray imaging device 10. A hashed arc-shaped arrow 35 indicates a further sliding movement, or roll movement. Fig. 4b shows a perspective view of an end posture *P_{E}* of the roll scan.

In an example, the angular length of the carrier, and guiding section in the base allow for a range of approximately 180° to be traversed. Hereby, the ends of the carrier do not surpass the ends of the guiding section in the base. Scanning over a larger angular range would result in a 3D reconstruction with less, or no obstructive artefacts. In an example, a larger scan range is realized by increasing the angular length of the carrier. In another alternative example, the length of the guiding section in the base is increased. Allowing the ends of the carrier to surpass the ends of this guiding section would result in a larger range of motion. In an example, however, the mechanical design of the roll guidance is optimized, by e.g. eliminating backlash. This leads to less excitation of dynamics due to transversal of backlash, which improves image quality. Furthermore, it results in a more linear mechanical behavior, and better motion reproducibility. Hereby, calibration time can potentially be reduced using e.g. model based calibration.

Also, shown as an option e.g. in Fig. 4b, the sliding carrier support 24 is provided with a guiding section part 34 that extends on one side of the base as an arc-segment.

In an example, not shown in detail, the guiding section part 34 comprises a bearing section of several bearings. The sliding movement of the C-arm 22 along the sliding carrier support 24 is limited to a range in which the C-arm is supported by all of the several bearings.

In an example, the bearing section is arranged in the distal part of the arc-segment of the guiding section part.

In an example, the bearing section is arranged as a moving bearing support that travels along the moving direction during the motion. In a further example, also not shown in detail, the guiding section part comprises a travelling bearing arrangement. The travelling bearing arrangement provides a bearing with bearings elements, such as rollers, that travel along in movement direction during movement.

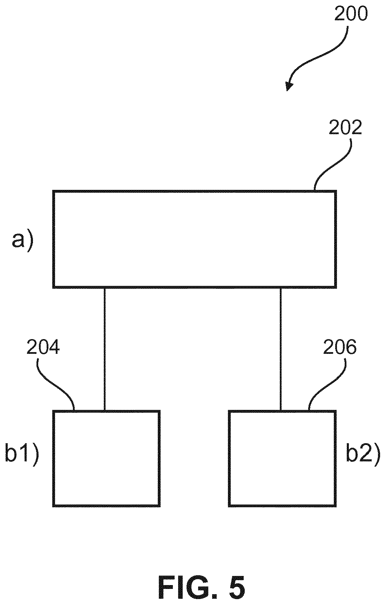

Fig. 5 shows a method 200 for movably supporting an X-ray imaging arrangement. The method comprises the following steps: In a first step, also referred to as step a), an X-ray imaging arrangement with an X-ray source and an X-ray detector is provided. The device has a support structure with a base configured to be rotatably attached to a fixed part of a building structure such that the base can be rotated in relation to the fixed part. The device also has a C-arm with two opposing ends, at which the X-ray source and the X-ray detector are mounted. The device further has a sliding carrier support with which the C-arm is movably mounted to the base. The support structure is movably carrying the X-ray imaging arrangement with a limitation of two degrees of freedom: The rotatably attached base provides a first degree of freedom in a first rotational manner, and the sliding carrier support provides a second degree of freedom such that the C-arm can be moved in relation to the sliding carrier support along an arc in a second rotational manner. The first rotational manner and the second rotational manner are provided transverse to each other; and the first rotational manner is provided around a vertical axis of rotation. In a first sub-step 204 of a second step, the first sub-step being also referred to as step b1), the base is rotatably moved around the first axis. In addition, or alternatively, in a second sub-step 206 of the second step, the second sub-step also referred to as step b2), the C-arm is slidingly moved within the sliding carrier support to rotatably move the C-arm around the second axis.

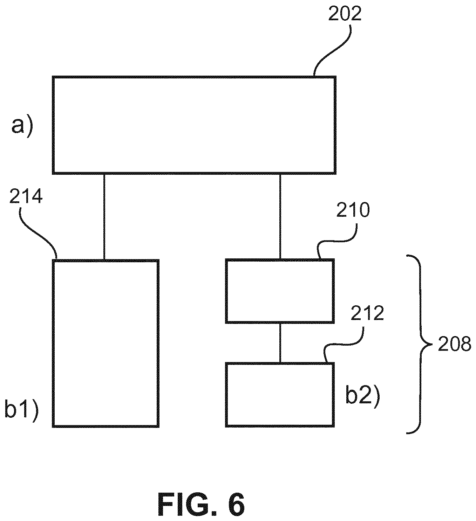

Fig. 6 shows a further example of the method of Fig. 5. In this further example, a dual axis movement 208 is provided, e.g. for a dual axis 3D scan. The dual axis movement 208 is indicated with a bracket encompassing the second step b) with its sub-steps b1) and b2). For step b2), the C-arm with the X-ray imaging arrangement is rotating in an initial rotation 210 in the sliding carrier support to a first angle around the second axis of rotation as a starting position; and subsequently rotating in a rotation 212 from this starting position to a near-neutral position in which an imaging viewing direction is approximately aligned with the first axis of rotation, and back to the starting position. In addition, for step b1), the base is rotating in a rotation 214 over an angle of approximately 180° around the first axis of rotation. These rotation motions are synchronized such that a moment of nearly zero roll velocity around the second axis of rotation corresponds to a rotation of the base around the first axis of rotation of approximately 90°.

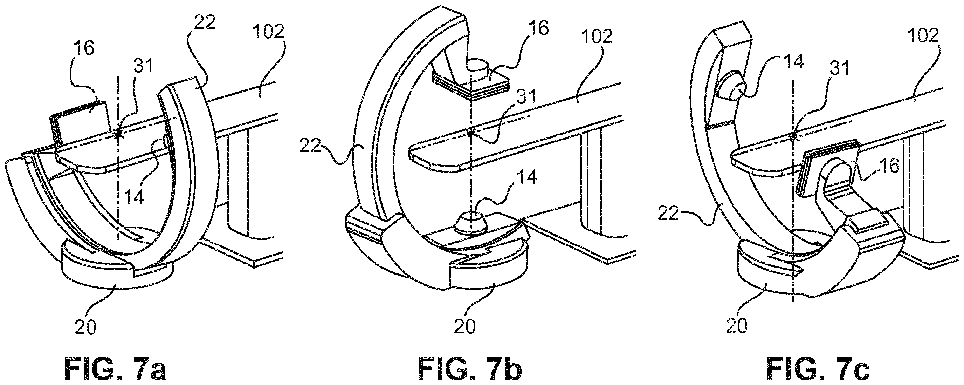

Figs. 7a, 7b and 7c show different postures, i.e. positions, during a dual axis 3D scan. The dual axis 3D scan is provided as an example of capturing a set of 2D images for a 3D reconstruction.
- This scan starts with the carrier 22 and imaging equipment, i.e. the X-ray source 14 and the X-ray detector rotated to a certain angle around the horizontal axis, for example 105°. Fig. 7a shows the begin or starting posture, i.e. starting position. The term "posture" relates to the spatial arrangement, i.e. the position of the equipment in relation to each other.
- The carrier 22 and the imaging equipment are rotated from this starting posture to near the neutral posture, i.e. the middle position. Fig. 7b shows an example of a middle posture of the dual axis 3D scan.
- The carrier 22 and the imaging equipment are then rotated from this middle posture back to the starting posture, i.e. starting position. Fig. 7c shows an example of an end posture of the dual axis 3D scan.

During this movement, the base is rotated over an angle of approximately 180° around the vertical axis. These motions are synchronized, such that the moment of zero roll velocity corresponds to a rotation of the base of approximately 90°. By using both rotational degrees of freedom (*DOFs*), the imaging equipment can be moved over a quasi-circular trajectory, with no points of zero velocity. The angular range of this trajectory, e.g. 210°, is larger than the range of motion of the carrier in the base, and the ends of the carrier do not have to surpass the ends of the guiding section in the base. Both aspects cause the dual axis 3D scan to allow having improved image quality over the 3D roll scan.

As an option, a scan trajectory for acquiring 2D images used in 3D computed tomography is provided, by combining a rotation around a nominally vertical axis with a roll motion. Synchronous rotation of the imaging equipment around two axes allows images to be gathered over a large angular range, e.g. approximately 210°, with only limited travel of the carrier in the base. Non-linear mechanical behavior, and excitation of dynamics are hereby minimized, in comparison to existing devices which carry out a large range rotation using only the roll motion. This, combined with the large scanning range, decreases reconstruction artefacts, and allows calibration time to be reduced.

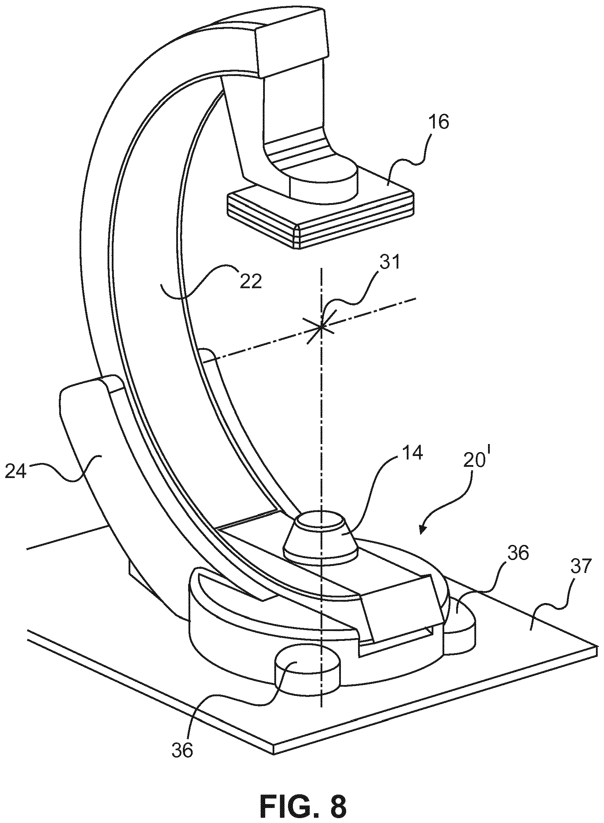

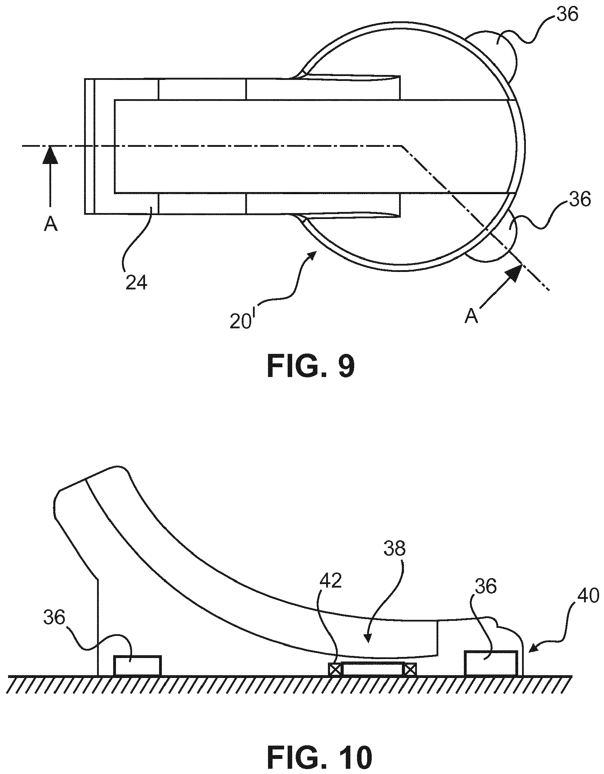

Fig. 8 shows another option, in which the base 20 is configured as a moveable floor base 20'. The moveable floor base 20' comprises a plurality of supporting modules 36 configured to provide support for the base in a vertical direction on a floor surface 37 and to allow a movement of the base 20' in horizontal direction. The moveable floor base 20' further comprises a central bearing module 38 (see Fig. 10) configured to provide releasable fixation of the base 20' in the horizontal plane of the floor while allowing rotational movement of the base. Further, a locking mechanism 40 (see Fig. 10) is provided configured to provide temporal fixation of the rotational movement of the base during imaging. For example, three of the supporting modules 36 are provided.

Fig. 9 shows a top view of the base of Fig. 8. Two supporting modules are indicated by extensions of a housing of the base 20', whereas a third of the supporting modules 36 is hidden below the sliding carrier support 24.

Fig. 10 shows a vertical cross section of the base along A-A in Fig. 9. In Fig. 10, two of the supporting modules 36 are indicated. Further, the central bearing module 38 is also shown. The central bearing module 38 provides rotation around a vertical rotation axis, which defines the first axis of rotation for the first rotational manner. The support modules 36 support the weight of the imaging arrangement, C-arm and base, while the central bearing module 38 provides for the rotational movement of the base around the vertical axis as the first axis of rotation.

For example, the supporting modules 36 are provided as at least one of the group of: wheels, air casters and magnetic bearings. In an example, the supporting modules are provided as wheels, e.g. omnidirectional wheels. In another example, the supporting modules are provided as air casters providing an air film for movably supporting the X-ray imaging arrangement. In a further example, the supporting modules are provided as magnetic bearings.

According to an example, a weight bearing pivot (radial and axial bearing) is provided on a moving module beneath the base. This module is configured to be held completely stationary during imaging.

In an example, the base is supported on the separate moving module. The bearing pivot can provide the rotation of the base around the vertical axis in relation to the moving module. While, during imaging, the movable module is held stationary, the base is not always held stationary by the bearing pivot, but rather moving, for example rotating around the vertical axis. The moving module is configured to be movable such that the moving module provides horizontal movement and thus allows horizontal movement of the base in relation to the floor, e.g. for moving the imaging arrangement to a parking position when not in use, or to a remote location for situations during an intervention when the imaging arrangement is temporarily not required.

In an option, the moving module is provided in combination with the moveable floor base. In an example, the moving module is provided for larger movements in relation to the floor such as for storing or parking purposes. In addition, for imaging, the moveable floor base with the plurality of supporting modules, the central bearing module and the locking mechanism is provided for smaller and more precise movements in relation to the floor, such as for precise aligning and positioning purposes. The base provides in particular the rotation around the vertical axis.

In another option, only the moveable floor base with the plurality of supporting modules, the central bearing module and the locking mechanism is provided. In an example, the moveable floor base is provided for larger movements in relation to the floor such as for storing or parking purposes, and also, for imaging, the moveable floor base is provided for smaller and more precise movements in relation to the floor, such as for precise aligning and positioning purposes. The base provides in particular the rotation around the vertical axis.

In an option, it is provided that at least one of the supporting modules 36 is temporarily lockable to prevent rotational movement during imaging.

In another option, provided in addition or alternatively, the central bearing module 38 is temporarily lockable to prevent the rotational movement during imaging.

The central bearing prevents horizontal translation movement.

In an example, the supporting modules are temporarily lockable as to provide the locking mechanism. In another example, the central bearing module is temporarily lockable as to provide the locking mechanism.

In another example, not shown, the locking mechanism is provided as a separate mechanism in addition to the supporting modules and the central bearing module. For example, lowerable pins are provided to provide the locking function.

In an example, indicated in Fig. 10, the central bearing module 38 is provided as a spindle attached to the base by a radial bearing 42. The spindle is temporarily attachable to a floor surface.

As an option, the spindle is attracted to the floor by an attractive force for attaching the spindle to the floor surface (not shown in detail). For example, the spindle is attracted to the floor by magnetic forces or by generating an underpressure, such as a vacuum or soft vacuum, between the spindle and the floor surface.

In an example, the central bearing module is provided as a pin that is insertable in a recess in the floor to provide fixation in the horizontal plane, while allowing rotational movement around an axis defined by the pin. For example, the pin is movably held by an electromagnetic actuator that can be controlled for lowering and raising the pin.

In another example, for locking rotational movements, locking elements are provided to interact with the floor, for example lowerable pins or magnetically activatable connectors.

In a still further example, pins are provided that can be extended upwardly from the floor to interact with the central bearing module.

In an example, for supplying the X-ray imaging arrangement, a cabling connection is provided from beneath a patient support table.

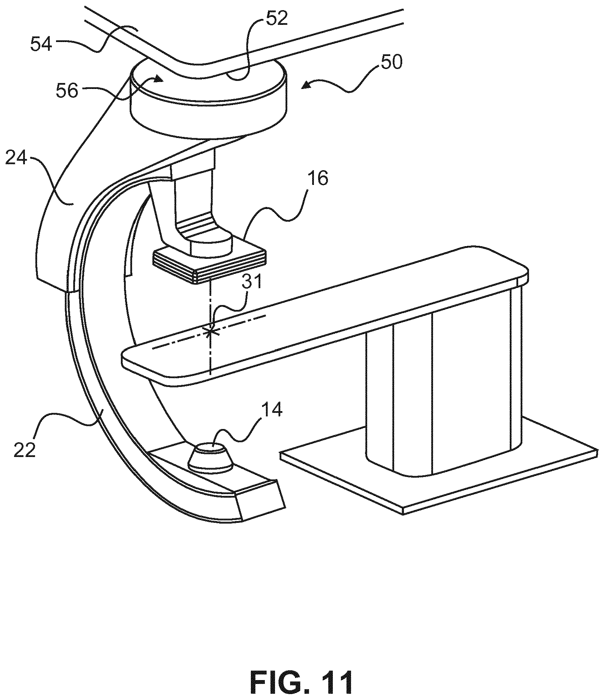

Fig. 11 shows an example of the X-ray imaging device 10 in which the base 20 is configured as a ceiling-attached support 50 that comprises a rotational joint 52 configured to be mounted to a fixed ceiling structure 54 and configured to allow a rotational movement of the base. Further, the ceiling-attached support 50 comprises a locking mechanism 56 (not shown in detail) configured to provide temporal fixation of the rotational movement of the base during imaging

The X-ray imaging device 10 with the ceiling-attached support 50 is further provided with the sliding carrier support 24 and the C-arm 22 with the X-ray source 14 and the X-ray detector 16.

In an example, not further shown in detail, the base comprises a carriage that is mounted to rails to allow movement in relation to the ceiling, for example for temporary storing or permanent parking purposes.

The device features the same degrees of freedom as that of the examples above. However, the base is mounted to the ceiling by a rotational joint. A ceiling mounted device could provide benefits in terms of room layout, cabling, etc., but would potentially introduce additional cost and requirements (height, structural integrity) for the medical room.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An X-ray imaging device (10), comprising:
- an X-ray imaging arrangement (12) with an X-ray source (14) and an X-ray detector (16);
- a support structure (18);
wherein the support structure comprises:
- a base (20) configured to be rotatably attached to a fixed part of a building structure such that the base can be rotated in relation to the fixed part; and
- a C-arm (22) with two opposing ends, at which the X-ray source and the X-ray detector are mounted; and
- a sliding carrier support (24) with which the C-arm is movably mounted to the base;
wherein the support structure is movably carrying the X-ray imaging arrangement with a limitation of two degrees of freedom:
i) wherein the rotatably attached base provides a first degree of freedom in a first rotational manner (26); and
ii) wherein the sliding carrier support provides a second degree of freedom such that the C-arm can be moved in relation to the sliding carrier support along an arc in a second rotational manner (28);
wherein the first rotational manner and the second rotational manner are provided transverse to each other; and
wherein the first rotational manner is provided around a vertical axis of rotation (30).

2. An X-ray imaging device according to claim 1, wherein the first rotational manner and the second rotational manner are arranged essentially perpendicular to each other; and
wherein the second rotational manner is provided around a horizontal axis of rotation (32).

3. An X-ray imaging device according to claim 1 or 2, wherein the sliding carrier support is provided with a guiding section part (34) that extends on one side of the base as an arc-segment.

4. An X-ray imaging device according to claim 3, wherein the guiding section part comprises a bearing section of several bearings; and
wherein the sliding movement of the C-arm along the sliding carrier support is limited to a range in which the C-arm is supported by all of the several bearings.

5. An X-ray imaging device according to claim 3 or 4, wherein the guiding section part comprises a travelling bearing arrangement.

6. An X-ray imaging device according to one of the preceding claims, wherein a radial and axial weight bearing pivot is provided on a moving module beneath the base; and
wherein the moving module is configured to be held completely stationary during imaging.

7. An X-ray imaging device according to one of the claims 1 to 5, wherein the base is configured as a moveable floor base that comprises:
- a plurality of supporting modules (36) configured to provide support for the base in a vertical direction on a floor surface and to allow a movement of the base in horizontal direction; and
- a central bearing module (38) configured to provide releasable fixation of the base in the horizontal plane of the floor while allowing rotational movement of the base; and
- a locking mechanism (40) configured to provide temporal fixation of the rotational movement of the base during imaging.

8. An X-ray imaging device according to claim 7, wherein the supporting modules are provided as at least one of the group of: wheels, air casters and magnetic bearings; and/or
wherein at least one of the supporting modules is temporarily lockable to prevent rotational movement during imaging; and/or
wherein the central bearing module is temporarily lockable to prevent the rotational movement during imaging.

9. An X-ray imaging device according to claim 7 or 8, wherein the central bearing module is provided as a spindle attached to the base by a radial bearing, which spindle is temporarily attachable to a floor surface.

10. An X-ray imaging device according to claim 9, wherein the spindle is attracted to the floor by an attractive force for attaching the spindle to the floor surface.

11. An X-ray imaging device according to one of the claims 1 to 5, wherein the base is configured as a ceiling-attached support that comprises:
- a rotational joint configured to be mounted to a fixed ceiling structure and configured to allow a rotational movement of the base; and
- a locking mechanism configured to provide temporal fixation of the rotational movement of the base during imaging.

12. An X-ray imaging system (100), comprising:
- an X-ray imaging device (10) according to one of the preceding claims; and
- a subject support (102);
wherein, during use, the base is arranged below or above the subject support.

13. A method (200) for movably supporting an X-ray arrangement, comprising the following steps:
a) providing (202) an X-ray imaging arrangement with an X-ray source and an X-ray detector, the device having a support structure with a base configured to be rotatably attached to a fixed part of a building structure such that the base can be rotated in relation to the fixed part, and a C-arm with two opposing ends, at which the X-ray source and the X-ray detector are mounted, and a sliding carrier support with which the C-arm is movably mounted to the base; wherein the support structure is movably carrying the X-ray imaging arrangement with a limitation of two degrees of freedom: wherein the rotatably attached base provides a first degree of freedom in a first rotational manner; and wherein the sliding carrier support provides a second degree of freedom such that the C-arm can be moved in relation to the sliding carrier support along an arc in a second rotational manner; wherein the first rotational manner and the second rotational manner are provided transverse to each other; and wherein the first rotational manner is provided around a vertical axis of rotation; and
b1) rotatably moving (204) the base around the first axis; and/or
b2) slidingly moving (206) the C-arm within the sliding carrier support to rotatably move the C-arm around the second axis.

14. Method according to claim 13, wherein a dual axis movement (208) is provided, wherein:
for step b2), the C-arm with the X-ray imaging arrangement is:
- rotating in an initial rotation (210) in the sliding carrier support to a first angle around the second axis of rotation as a starting position; and
- rotating in a rotation (212) from this starting position to a near-neutral position in which an imaging viewing direction is approximately aligned with the first axis of rotation, and back to the starting position;
for step b1), the base is rotating in a rotation (214) over an angle of approximately 180° around the first axis of rotation; and
wherein the two rotation motions are synchronized such that a moment of nearly zero roll velocity around the second axis of rotation corresponds to a rotation of the base around the first axis of rotation of approximately 90°.
